# EUROPEAN PATENT SPECIFICATION

(11) **EP 3 146 715 B1**
(45) Date of publication and mention of the grant of the patent: **23.03.2022**
(21) Application number: 15795790.3
(22) Date of filing: 19.05.2015
(51) Int. Cl.: G06F 3/01, H04N 5/225, A61B 1/00, A61B 1/045, A61B 1/05, A61B 34/20, A61B 90/30, H04N 13/111, H04N 13/156, H04N 13/239, H04N 13/344

(54) **SYSTEMS AND METHODS FOR MEDIATED-REALITY SURGICAL VISUALIZATION**
SYSTEME UND VERFAHREN ZUR CHIRURGISCHEN VISUALISIERUNG MIT VERMITTELTER REALITÄT
SYSTÈMES ET PROCÉDÉS DE VISUALISATION CHIRURGICALE PAR RÉALITÉ INDUITE

(30) Priority: 20.05.2014 US 201462000900 P
(43) Date of publication of application: 29.03.2017
(73) Proprietor: University of Washington through its Center for Commercialization, Seattle, WA 98105-4608 (US)
(72) Inventor: BROWD, Samuel R., Seattle, WA 98105-4608 (US); SMITH, Joshua R., Seattle, WA 98105-4608 (US); NICOLL, Rufus Griffin, Seattle, WA 98105-4608 (US)
(74) Representative: Forresters IP LLP
(86) International application number: PCT/US2015/031637
(87) International publication number: WO 2015/179446

(56) References cited:
- EP-A1- 1 027 627
- WO-A1-01/05161
- WO-A2-2014/037953
- US-A1- 2005 090 730
- US-A1- 2005 203 380
- US-A1- 2007 121 423
- US-A1- 2007 236 514
- US-A1- 2010 045 783
- US-A1- 2013 265 485

## Description

### TECHNICAL FIELD

The present technology is generally related to mediated-reality surgical visualization and associated systems and methods. In particular, several embodiments are directed to head-mounted displays configured to provide mediated-reality output to a wearer for use in surgical applications.

### BACKGROUND

The history of surgical loupes dates back to 1876. Surgical loupes are commonly used in neurosurgery, plastic surgery, cardiac surgery, orthopedic surgery, and microvascular surgery. Despite revolutionary change in virtually every other point of interaction between surgeon and patient, the state of the art of surgical visual aids has remained largely unchanged since their inception. Traditional surgical loupes, for example, are mounted in the lenses of glasses and are custom made for the individual surgeon, taking into account the surgeon's corrected vision, interpupillary distance, and a desired focal distance. The most important function of traditional surgical loupes is their ability to magnify the operative field and empower the surgeon to perform maneuvers at a higher level of precision than would otherwise be possible.

Traditional surgical loupes suffer from a number of drawbacks. They are customized for each individual surgeon, based on the surgeon's corrective vision requirements and interpupillary distance, and so cannot be shared among surgeons. Traditional surgical loupes are also restricted to a single level of magnification, forcing the surgeon to adapt all of her actions to that level of magnification, or to frequently look "outside" the loupes at odd angles to perform actions where magnification is unhelpful or even detrimental. Traditional loupes provide a sharp image only within a very shallow depth of field, while also offering a relatively narrow field of view. Blind spots are another problem, due to the bulky construction of traditional surgical loupes.

US2010/045783 discloses methods and systems for dynamic virtual convergence and a video see through head mountable display that uses dynamic virtual convergence are disclosed. A dynamic virtual convergence algorithm includes sampling an image with two cameras. The cameras each have a field of view that is larger than a field of view of displays used to display images sampled by the cameras. A heuristic is used to estimate the gaze distance of the viewer. The display frustums are transformed so that they converge at the estimated gaze distance. The images sampled by the cameras are then reprojected into the transformed display frustums. The reprojected images are displayed to the user to simulate viewing of close range objects.

US2005/203380 discloses a method for augmented reality navigation of a medical intervention includes providing a stereoscopic head mounted display, the display including a pair of stereo viewing cameras, at least one tracking camera, and a stereoscopic guidance display. During a medical intervention on a patient, the patient's body pose is determined from a rigid body transformation between the tracking camera and frame markers on the scanning table, and the pose of an intervention instrument with respect to the table is determined. A visual representation of the patient overlaid with an image of the intervention target, the instrument, and a path for guiding the instrument to perform said medical intervention is displayed in the stereoscopic guidance display.

EP1027627 discloses eyeglass interface system which integrates interface systems within eyewear. The system includes a display assembly and one or more audio and/or video assemblies mounted to an eyeglass frame. The display assembly is mounted to one temple and provides an image which can be viewed by the user. The audio or video assembly is mounted to the other temple and is in communication with the display assembly. The audio or video assembly may comprise such as microphone and/or speakers.

US2013/265485 discloses a plenoptic camera apparatus includes a main lens unit configured to collect rays emitted from a subject. The plenoptic camera apparatus includes an image sensor unit adapted to capture images formed from the collected rays through one or more convertible lens device units. The convertible lens device units are positioned between the main lens unit and the image sensor unit and configured to provide individual curvature adjustment. The plenoptic camera apparatus includes a control unit configured to control the convertible lens device units to switch to a high-resolution imaging lens or a plenoptic imaging lens array, based on a user's selection, and control driving of the convertible lens device units to compensate for a partial aberration caused by the main lens unit.

### BRIEF DESCRIPTION OF THE DRAWINGS

Figure 1A is a front perspective view of a head-mounted display assembly with an integrated imaging device.
Figure 1B is a rear perspective view of the head-mounted display of Figure 1A.
Figure 2 is a schematic representation of a mediated-reality surgical visualization system configured in accordance with an embodiment of the present technology.
Figure 3 illustrates a mediated-reality surgical visualization system in operation.
Figures 4A-4I are schematic illustrations of plenoptic cameras configured for use in a mediated-reality surgical visualization system in accordance with embodiments of the present technology.
Figure 5 is a block diagram of a method for providing a mediated-reality display for surgical visualization according to one embodiment of the present technology.

### DETAILED DESCRIPTION

The present technology is directed to systems and methods for providing mediated-reality surgical visualization. In one embodiment, for example, a head-mounted display assembly can include a stereoscopic display device configured to display a three-dimensional image to a user wearing the assembly. An imaging device can be coupled to the head-mounted display assembly and configured to capture images to be displayed to the user. Additional image data from other imagers can be incorporated or synthesized into the display. As used herein, the term "mediated-reality" refers to the ability to add to, subtract from, or otherwise manipulate the perception of reality through the use of a wearable display. "Mediated reality" display includes at least "virtual reality" as well as "augmented reality" type displays.

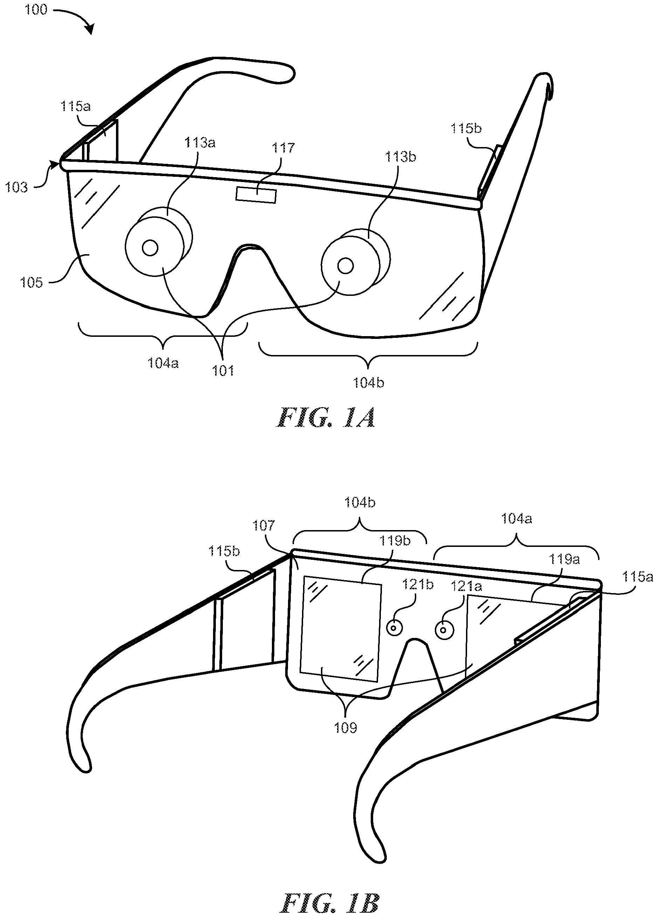

Specific details of several embodiments of the present technology are described below with reference to Figures 1A-5. Although many of the embodiments are described below with respect to devices, systems, and methods for managing multiple mediated-reality surgical visualization, other embodiments are within the scope of the present technology. Additionally, other embodiments of the present technology can have different configurations, components, and/or procedures than those described herein. For instance, other embodiments can include additional elements and features beyond those described herein, or other embodiments may not include several of the elements and features shown and described herein.

For ease of reference, throughout this disclosure identical reference numbers are used to identify similar or analogous components or features, but the use of the same reference number does not imply that the parts should be construed to be identical. Indeed, in many examples described herein, the identically numbered parts are distinct in structure and/or function.

### Selected Embodiments of Mediated-Reality Surgical Visualization Systems

Figures 1A and 1B are front perspective and rear perspective views, respectively, of a head-mounted display assembly 100 with an integrated imaging device 101. The assembly 100 comprises a frame 103 having a forward surface 105 and a rearward surface 107 opposite the forward surface 105. The imaging device 101 is disposed over the forward surface 105 and faces forward. A display device 109 is disposed over the rearward surface 107 and outwardly away from the rearward surface 107 (and in a direction opposite to the imaging device 101). The assembly 100 is generally configured to be worn over a user's head (not shown), and in particular over a user's eyes such that the display device 109 displays an image towards the user's eyes.

In the illustrated embodiment, the frame 103 is formed generally similar to standard eyewear, with orbitals joined by a bridge and temple arms extending rearwardly to engage a wearer's ears. In other embodiments, the frame 103 can assume other forms; for example, a strap can replace the temple arms or, in some embodiments, a partial helmet can be used to mount the assembly 100 to a wearer's head. The frame 103 includes a right-eye portion 104a and a left-eye portion 104b. When worn by a user, the right-eye portion 104a is configured to generally be positioned over a user's right eye, while the left-eye portion 104b is configured to generally be positioned over a user's left eye. The assembly 100 can generally be opaque, such that a user wearing the assembly 100 will be unable to see through the frame 103. In other embodiments, however, the assembly 100 can be transparent or semitransparent, so that a user can see through the frame 103 while wearing the assembly 100. The assembly 100 can be configured to be worn over a user's standard eyeglasses. The assembly 100 can include tempered glass or other sufficiently sturdy material to meet OSHA regulations for eye protection in the surgical operating room.

The imaging device 101 includes a first imager 113a and a second imager 113b. The first and second imagers 113a-b can be, for example, digital video cameras such as CCD or CMOS image sensor and associated optics. In some embodiments, each of the imagers 113a-b can include an array of cameras having different optics (e.g., differing magnification factors). The particular camera of the array can be selected for active viewing based on the user's desired viewing parameters. In some embodiments, intermediate zoom levels between those provided by the separate cameras themselves can be computed. For example, if a zoom level of 4.0 is desired, an image captured from a 4.6 magnification camera can be down-sampled to provide a new, smaller image with this level of magnification. However, now this image may not fill the entire field of view of the camera. An image from a lower magnification camera (e.g., a 3.3 magnification image) has a wider field of view, and may be up-sampled to fill in the outer portions of the desired 4.0 magnification image. In another embodiment, features from a first camera (such as a 3.3 magnification camera) may be matched with features from the second camera (e.g., a 4.6 magnification camera). To perform the matching, features such as SIFT or SURF may be used. With features from different images matched, the different images captured with different levels of magnification can be combined more effectively and in a fashion that introduces less distortion and error. In another embodiment, each camera may be equipped with a lenslet array between the image sensor and the main lens. This lenslet array allows capture of "light fields," from which images with different focus planes and different viewpoints (parallax) can be computed. Using light field parallax adjustment techniques, differences in image point of view between the various cameras can be compensated away, so that as the zoom level changes, the point of view does not. In another embodiment, so-called "origami lenses," or annular folded optics, can be used to provide high magnification with low weight and volume.

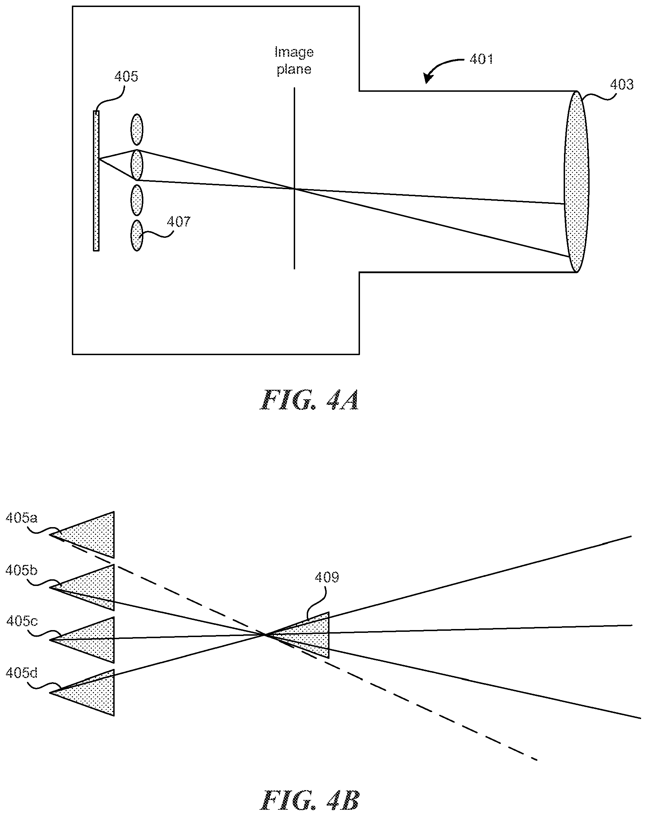

The first and second imagers 113a-b include one or more plenoptic cameras (also referred to as light field cameras). For example, instead of multiple lenses with different degrees of magnification, a plenoptic camera alone may be used for each imager. The first and second imagers 113a-b can each include a single plenoptic camera: a lens, a lenslet array, and an image sensor. By sampling the light field appropriately, images with varying degrees of magnification can be extracted. In some embodiments, a single plenoptic camera can be utilized to simulate two separate imagers from within the plenoptic camera. The use of plenoptic cameras is described in more detail below with respect to Figures 4A-I.

The first imager 113a is disposed over the right-eye portion 104a of the frame 103, while the second imager 113b is disposed over the left-eye portion 104b of the frame 103. The first and second imagers 113a-b are oriented forwardly such that when the assembly 100 is worn by a user, the first and second imagers 113a-b can capture video in the natural field of view of the user. For example, given a user's head position when wearing the assembly 100, she would naturally have a certain field of view when her eyes are looking straight ahead. The first and second imagers 113a-b can be oriented so as to capture this field of view or a similar field of view when the user dons the assembly 100. In other embodiments, the first and second imagers 113a-b can be oriented to capture a modified field of view. For example, when a user wearing the assembly 100 rests in a neutral position, the imagers 113a-b may be configured to capture a downwardly oriented field of view.

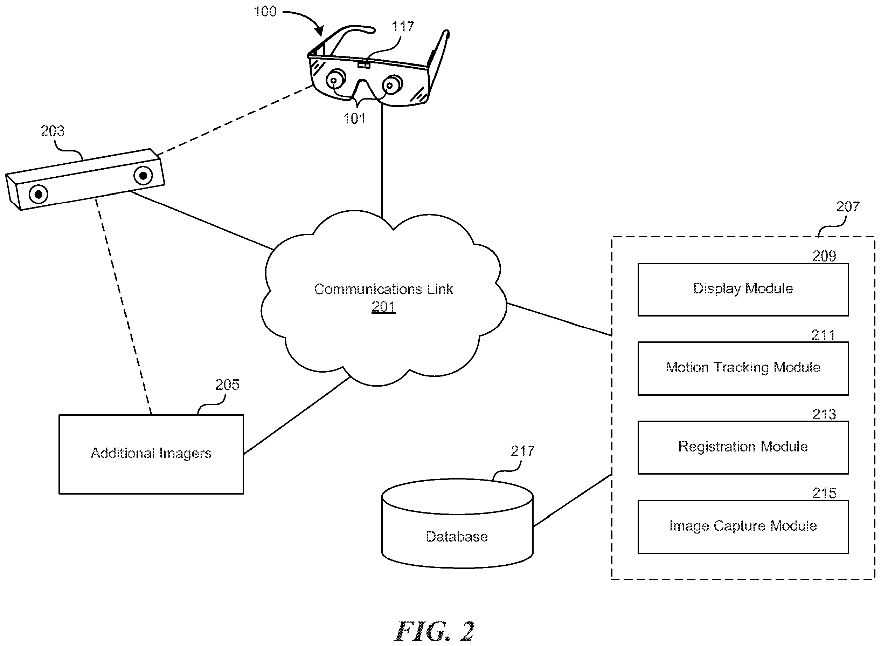

The first and second imagers 113a-b can be electrically coupled to first and second control electronics 115a-b, respectively. The control electronics 115a-b can include, for example, a microprocessor chip or other suitable electronics for receiving data output from and providing control input to the first and second imagers 113a-b. The control electronics 115a-b can also be configured to provide wired or wireless communication over a network with other components, as described in more detail below with respect to Figure 2. In the illustrated embodiment, the control electronics 115a-b are coupled to the frame 103. In other embodiments, however, the control electronics 115a-b can be integrated into a single component or chip, and in some embodiments the control electronics 115a-b are not physically attached to the frame 103. The control electronics 115a-b can be configured to receive data output from the respective imagers 113a-b, and can also be configured to control operation of the imagers 113a-b (e.g., to initiate imaging, to control a physical zoom, autofocus, and/or to operate an integrated lighting source). In some embodiments, the control electronics 115a-b can be configured to process the data output from the imagers 113a-b, for example, to provide a digital zoom, to autofocus, and to adjust image parameters such as saturation, brightness, etc. In other embodiments, image processing can be performed on external devices and communicated to the control electronics 115a-b via a wired or wireless communication link. As described in more detail below, output from the imagers 113a-b can be processed to integrate additional data such as pre-existing images (e.g., X-ray images, fluoroscopy, MRI or CT scans, anatomical diagram data, etc.), other images being simultaneously captured (e.g., by endoscopes or other images disposed around the surgical site), patient vital data, etc. Additionally, further manipulation can allow for selective enlargement of regions within the field of view, as described in more detail below with respect to Figures 4A-I.

A fiducial marker 117 can be disposed over the forward surface 105 of the frame 103. The fiducial marker 117 can be used for motion tracking of the assembly 100. In some embodiments, for example, the fiducial marker 117 can be one or more infrared light sources that are detected by an infrared-light camera system. In other embodiments, the fiducial marker 117 can be a magnetic or electromagnetic probe, a reflective element, or any other component that can be used to track the position of the assembly 100 in space. The fiducial marker 117 can include or be coupled to an internal compass and/or accelerometer for tracking movement and orientation of the assembly 100.

On the rearward surface 107 of the frame 103, a display device 109 is disposed and faces rearwardly. As best seen in Figure 1B, the display device 109 includes first and second displays 119a-b. The displays 119a-b can include, for example, LCD screens, holographic displays, plasma screens, projection displays, or any other kind of display having a relatively thin form factor that can be used in a heads-up display environment. The first display 119a is disposed within the right-eye portion 104a of the frame 103, while the second display 119b is disposed within the left-eye portion 104b of the frame 103. The first and second displays 119a-b are oriented rearwardly such that when the assembly 100 is worn by a user, the first and second displays 119a-b are viewable by the user with the user's right and left eyes, respectively. The use of a separate display for each eye allows for stereoscopic display. Stereoscopic display involves presenting slightly different 2-dimensional images separately to the left eye and the right eye. Because of the offset between the two images, the user perceives 3-dimensional depth.

The first and second displays 119a-b can be electrically coupled to the first and second control electronics 115a-b, respectively. The control electronics 115a-b can be configured to provide input to and to control operation of the displays 119a-b. The control electronics 115a-b can be configured to provide a display input to the displays 119a-b, for example, processed image data that has been obtained from the imagers 113a-b. For example, in in one embodiment image data from the first imager 113a is communicated to the first display 119a via the first control electronics 115a, and similarly, image data from the second imager 113b is communicated to the second display 119b via the second control electronics 115b. Depending on the position and configuration of the imagers 113a-b and the displays 119a-b, the user can be presented with a stereoscopic image that mimics what the user would see without wearing the assembly 100. In some embodiments, the image data obtained from the imagers 113a-b can be processed, for example, digitally zoomed, so that the user is presented with a zoomed view via the displays 119a-b.

First and second eye trackers 121a-b are disposed over the rearward surface 107 of the frame 103, adjacent to the first and second displays 119a-b. The first eye tracker 121a can be positioned within the right-eye portion 104a of the frame 103, and can be oriented and configured to track the movement of a user's right eye while a user wears the assembly 100. Similarly, the second eye tracker 121b can be positioned within the left-eye portion 104b of the frame 103, and can be oriented and configured to track the movement of a user's left eye while a user wears the assembly 100. The first and second eye trackers 121a-b can be configured to determine movement of a user's eyes and can communicate electronically with the control electronics 115a-b. In some embodiments, the user's eye movement can be used to provide input control to the control electronics 115a-b. For example, a visual menu can be overlaid over a portion of the image displayed to the user via the displays 119a-b. A user can indicate selection of an item from the menu by focusing her eyes on that item. Eye trackers 121a-b can determine the item that the user is focusing on, and can provide this indication of item selection to the control electronics 115a-b. For example, this feature allows a user to control the level of zoom applied to particular images. In some embodiments, a microphone or physical button(s) can be present on the assembly 100, and can receive user input either via spoken commands or physical contact with buttons. In other embodiments other forms of input can be used, such as gesture recognition via the imagers 113a-b, assistant control, etc.

The technology described herein may be applied to endoscope systems. For example, rather than mounting the multiple cameras (with different field or view/magnification combinations) on the user's forehead, the multiple cameras may be mounted on the tip of the endoscopic instrument. Alternatively, a single main lens plus a lenslet array may be mounted on the tip of the endoscopic instrument. Then light field rendering techniques such as refocusing, rendering stereo images from two different perspectives, or zooming may be applied. In such cases, the collected images may be displayed through the wearable head-mounted display assembly 100.

Figure 2 is a schematic representation of a mediated-reality surgical visualization system configured in accordance with an embodiment of the present technology. The system includes a number of components in communication with one another via a communication link 201 which can be, for example, a public internet, private network such as an intranet, or other network. Connection between each component and the communication link 201 can be wireless (e.g., WiFi, Bluetooth, NFC, GSM, cellular communication such as CDMA, 3G, or 4G, etc.) or wired (e.g., Ethernet, FireWire cable, USB cable, etc.). The head-mounted display assembly 100 is coupled to the communication link 201. In some embodiments, the assembly 100 can be configured to capture images via imaging device 101 and to display images to a user wearing the assembly via integrated display device 109. The assembly 100 additionally includes a fiducial marker 117 that can be tracked by a tracker 203. The tracker 203 can determine the position and movement of the fiducial marker 117 via optical tracking, sonic or electromagnetic detection, or any other suitable approach to position tracking. In some embodiments, the tracker 203 can be configured to use during surgery to track the position of the patient and certain anatomical features. For example, the tracker 203 can be part of a surgical navigation system such as Medtronic's StealthStation^{®} surgical navigation system. Such systems can identify the position of probes around the surgical site and can also interface with other intraoperative imaging systems such as MRI, CT, fluoroscopy, etc. The tracker 203 can also track the position of additional imagers 205, for example, other cameras on articulated arms around the surgical site, endoscopes, cameras mounted on retractors, etc. For example, the additional imagers 205 can likewise be equipped with probes or fiducial markers to allow the tracker 203 to detect position and orientation. The position information obtained by the tracker 203 can be used to determine the position and orientation of the additional imagers 205 with respect to the assembly 100 and with respect to the surgical site. In some embodiments, the additional imagers 205 can be selectively activated depending on the position and/or operation of the head-mounted display assembly 100. For example, when a user wearing the assembly 100 is looking at a certain area that is within the field of view of an additional imager 205, that additional imager 205 can be activated and the data can be recorded for synthesis with image data from the assembly 100. In some embodiments, the additional imagers 205 can be controlled to change their position and/or orientation depending on the position and/or operation of the head-mounted display assembly 100, for example by rotating an additional imager 205 to capture a field of view that overlaps with the field of view of the assembly 100.

A computing component 207 includes a plurality of modules for interacting with the other components via communication link 201. The computing component 207 includes, for example, a display module 209, a motion tracking module 211, a registration module 213, and an image capture module 215. In some embodiments, the computing component 207 can include a processor such as a CPU which can perform operations in accordance with computer-executable instructions stored on a computer-readable medium. In some embodiments, the display module, motion tracking module, registration module, and image capture module may each be implemented in separate computing devices each having a processor configured to perform operations. In some embodiments, two or more of these modules can be contained in a single computing device. The computing component 207 is also in communication with a database 217.

The display module 209 can be configured to provide display output information to the assembly 100 for presentation to the user via the display device 109. As noted above, this can include stereoscopic display, in which different images are provided to each eye via first and second display devices 119a-b (Figure 1B). The display output provided to the assembly 100 can include a real-time or near-real-time feed of video captured by the imaging device 101 of the assembly 100. In some embodiments, the display output can include integration of other data, for example, pre-operative image data (e.g., CT, MRI, X-ray, fluoroscopy), standard anatomical images (e.g., textbook anatomical diagrams or cadaver-derived images), or current patient vital signs (e.g., EKG, EEG, SSEP, MEP). This additional data can be stored, for example, in the database 217 for access by the computing component 207. In some embodiments, additional real-time image data can be obtained from the additional imagers 205 and presented to a user via display device 109 of the assembly 100 (e.g., real-time image data from other cameras on articulated arms around the surgical site, endoscopes, cameras mounted on retractors, etc.). Such additional data can be integrated for display; for example, it can be provided as a picture-in-picture or other overlay over the display of the real-time images from the imaging device 101. In some embodiments, the additional data can be integrated into the display of the real-time images from the imaging device 101; for example, X-ray data can be integrated into the display such that the user views both real-time images from the imaging device 101a and X-ray data together as a unified image. In order for the additional image data (e.g., X-ray, MRI, etc.) to be presented coherently with the real-time feed from the imaging device 101, the additional image data can be processed and manipulated based on the position and orientation of the assembly 100. Similarly, in some embodiments textbook anatomical diagrams or other reference images (e.g., labeled images derived from cadavers) can be manipulated and warped so as to be correctly oriented onto the captured image. This can enable a surgeon, during operation, to visualize anatomical labels from preexisting images that are superimposed on top of real-time image data. In some embodiments, the user can toggle between different views via voice command, eye movement to select a menu item, assistant control, or other input. For example, a user can toggle between a real-time feed of images from the imaging devices 101 and a real-time feed of images captured from one or more additional imagers 205.

The motion tracking module 211 can be configured to determine the position and orientation of the assembly 100 as well as any additional imagers 205, with respect to the surgical site. As noted above, the tracker 203 can track the position of the assembly 100 and additional imagers 205 optically or via other techniques. This position and orientation data can be used to provide appropriate display output via display module 209.

The registration module 213 can be configured to register all image data in the surgical frame. For example, position and orientation data for the assembly 100 and additional imagers 205 can be received from the motion tracking module 211. Additional image data, for example, pre-operative images, can be received from the database 217 or from another source. The additional image data (e.g., X-ray, MRI, CT, fluoroscopy, anatomical diagrams, etc.) will typically not have been recorded from the perspective of either the assembly 100 or of any of the additional imagers 205. As a result, the supplemental image data must be processed and manipulated to be presented to the user via display device 109 of the assembly 100 with the appropriate perspective. The registration module 213 can register the supplemental image data in the surgical frame of reference by comparing anatomical or artificial fiducial markers as detected in the pre-operative images and those same anatomical or artificial fiducial markers as detected by the surgical navigation system, the assembly 100, or other additional imagers 205.

The image capture module 215 can be configured to capture image data from the imaging device 101 of the assembly 100 and also from any additional imagers 205. The images captured can include continuous streaming video and/or still images. The imaging device 101, and optionally also one or more of the additional imagers 205, are plenoptic camerasand the image capture module 215 can be configured to receive the light field data and to process the data to render particular images. Such image processing for plenoptic cameras is described in more detail below with respect to Figures 4A-I.

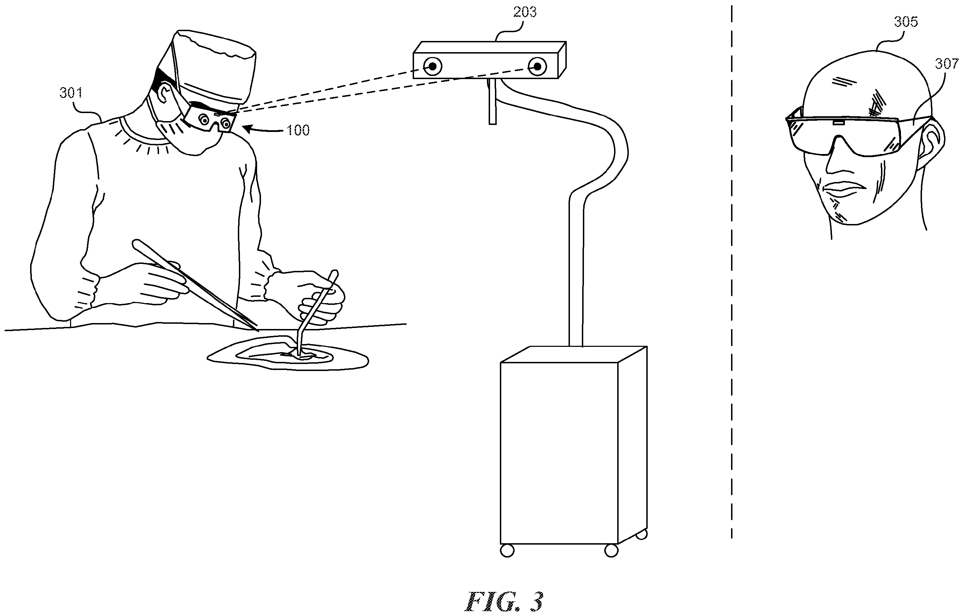

Figure 3 illustrates a mediated-reality surgical visualization system in operation. A surgeon 301 wears the head-mounted display assembly 100 during operation on a surgical site 303 of a patient. The tracker 203 follows the movement and position of the assembly 100. As noted above, the tracker 203 can determine the position and movement of the fiducial marker on the assembly 100 via optical tracking, sonic or electromagnetic detection, or any other suitable approach to position tracking. In some embodiments, the tracker 203 can be part of a surgical navigation system such as Medtronic's StealthStation^{®} surgical navigation system. The tracker 203 can also track the position of additional imagers, for example, other cameras on articulated arms around the surgical site, endoscopes, cameras mounted on retractors, etc.

While the surgeon 301 is operating, images captured via the imaging device 101 of the assembly 100 are processed and displayed stereoscopically to the surgeon via an integrated display device 109 (Figure 1B) within the assembly 100. The result is a mediated-reality representation of the surgeon's field of view. As noted above, additional image data or other data can be integrated and displayed to the surgeon as well. The display data being presented to the surgeon 301 can be streamed to a remote user 305, either simultaneously in real time or at a time delay. The remote user 305 can likewise don a head-mounted display assembly 307 configured with integrated stereoscopic display, or the display data can be presented to the remote user 305 via an external display. In some embodiments, the remote user 305 can control a surgical robot remotely, allowing telesurgery to be performed while providing the remote user 305 with the sense of presence and perspective to improve the surgical visualization. In some embodiments, multiple remote users can simultaneously view the surgical site from different viewpoints as rendered from multiple different plenoptic cameras and other imaging devices disposed around the surgical site.

The assembly 100 may respond to voice commands or even track the surgeon's eyes—thus enabling the surgeon 301 to switch between feeds and tweak the level of magnification being employed. A heads-up display with the patient's vital signs (EKG, EEG, SSEPs, MEPs), imaging (CT, MRI, etc.), and any other information the surgeon desires may scroll at the surgeon's request, eliminating the need to interrupt the flow of the operation to assess external monitors or query the anesthesia team. Wireless networking may infuse the assembly 100 with the ability to communicate with processors (e.g., the computing component 207) that can augment the visual work environment for the surgeon with everything from simple tools like autofocus to fluorescence video angiography and tumor "paint." The assembly 100 can replace the need for expensive surgical microscopes and even the remote robotic workstations of the near future—presenting an economical alternative to the current system of "bespoke" glass loupes used in conjunction with microscopes and endoscopes.

The head-mounted display assembly 100 can aggregate multiple streams of visual information and send it not just to the surgeon for visualization, but to remote processing power (e.g., the computing component 207 (Figure 2)) for real-time analysis and modification. In some embodiments, the system can utilize pattern recognition to assist in identification of anatomical structures and sources of bleeding requiring attention, thus acting as a digital surgical assistant. Real-time overlay of textbook or adaptive anatomy may assist in identifying structures and/or act as a teaching aid to resident physicians and other learners. In some embodiments, the system can be equipped with additional technology for interacting with the surgical field; for example, the assembly 100 can include LiDAR that may assist in analyzing tissue properties or mapping the surgical field in real time, thus assisting the surgeon in making decisions about extent of resection, etc. In some embodiments, the assembly 100 can be integrated with a high-intensity LED headlamp that can be "taught" (e.g., via machine-learning techniques) how to best illuminate certain operative situations or provide a different wavelength of light to interact with bio-fluorescent agents.

In some embodiments, the data recorded from the imaging device 101 and other imagers can be used to later generate different viewpoints and visualizations of the surgical site. For example, for later playback of the recorded data, an image having a different magnification, different integration of additional image data, and/or a different point of view can be generated. This can be particularly useful for review of the procedure or for training purposes.

Figures 4A-4I are schematic illustrations of plenoptic cameras configured for use in a mediated-reality surgical visualization system in accordance with embodiments of the present technology. As described above, one or more plenoptic cameras are used as the first and second imagers 113a-b coupled to the head-mounted display assembly 100. By processing the light fields captured with the plenoptic camera(s), images with different focus planes and different viewpoints can be computed.

Referring first to Figure 4A, a plenoptic camera 401 includes a main lens 403, an image sensor 405, and an array of microlenses or lenslets 407 disposed therebetween. Light focused by the main lens 403 intersects at the image plane and passes to the lenslets 407, where it is focused to a point on the sensor 405. The array of lenslets 407 results in capturing a number of different images from slightly different positions and, therefore, different perspectives. By processing these multiple images, composite images from varying viewpoints and focal lengths can be extracted to reach a certain depth of field. In some embodiments, the array of lenslets 407 and associated sensor 405 can be substituted for an array of individual separate cameras.

Figure 4B is a schematic illustration of rendering of a virtual camera using a plenoptic camera. An array of sensor elements 405 (four are shown as sensor elements 405a-d) correspond to different portions of the sensor 405 that receive light from different lenslets 407 (Figure 4A). The virtual camera 409 indicates the point of view to be rendered by processing image data captured via the plenoptic camera. Here the virtual camera 409 is "positioned" in front of the sensor elements 405a-d. To render the virtual camera 409, only light that would have passed through that position is used to generate the resulting image. As illustrated, virtual camera 409 is outside of the "field of view" of the sensor element 405a, and accordingly data from the sensor element 405a is not used to render the image from the virtual camera 409. The virtual camera 409 does fall within the "field of view" of the other sensor elements 405b-d, and accordingly data from these sensor elements 405b-d are combined to generate the image from the rendered virtual camera. It will be appreciated that although only four sensor elements 405a-d are shown, the array may include a different number of sensor elements 405.

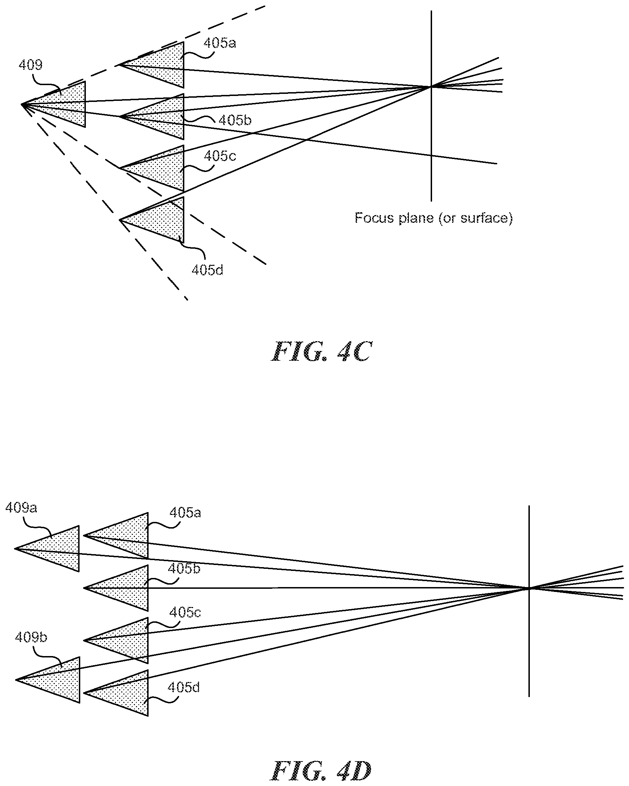

Figure 4C illustrates a similar rendering of a virtual camera but with the "position" of the virtual camera being behind the sensor elements 405a-d. Here the sensor elements 405a, c, and d are outside the "field of view" of the virtual camera 409, so data from these sensor elements are not used to render the image from the virtual camera 409. With respect to Figure 4D, two separate virtual cameras 409a and 409b are rendered using data from sensor elements 405a-d. This configuration can be used to generate two "virtual cameras" that would correspond to the position of a user's eyes when wearing the head-mounted display assembly 100. For example, a user wearing the assembly 100 would have the imaging device 101 disposed in front of her eyes. The sensor elements 405a-d (as part of the imaging device 101) are also disposed in front of the user's eyes. By rendering virtual cameras 409a-b in a position behind the sensor elements 405a-d, the virtual cameras 409a-b can be rendered at positions corresponding to the user's left and right eyes. The use of eye trackers 121a-b (Figure 1B) can be used to determine the lateral position of the user's eyes and interpupillary distance. This allows a single hardware configuration to be customized via software for a variety of different interpupillary distances for various different users. In some embodiments, the interpupillary distance can be input by the user rather than being detected by eye trackers 121a-b.

The use of plenoptic cameras can also allow the system to reduce perceived latency as the assembly moves and captures a new field of view. Plenoptic cameras can capture and transmit information to form a spatial buffer around each virtual camera. During movement, the local virtual cameras can be moved into the spatial buffer regions without waiting for remote sensing to receive commands, physically move to the desired location, and send new image data. As a result, the physical scene objects captured by the moved virtual cameras will have some latency, but the viewpoint latency can be significantly reduced.

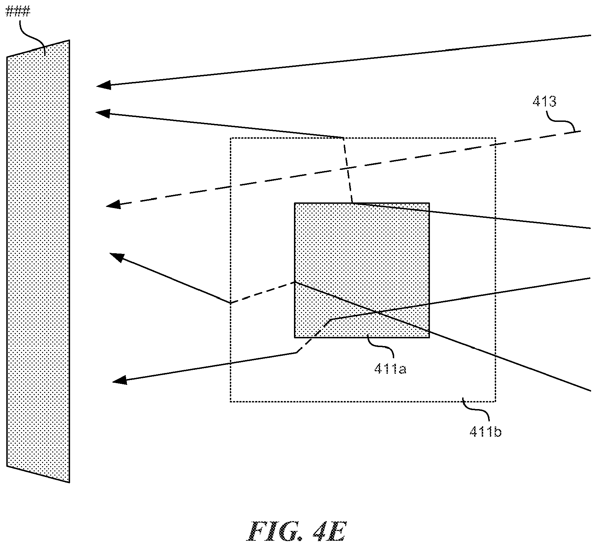

Figure 4E is a schematic illustration of enlargement using a plenoptic camera. Area 411a indicates a region of interest to be enlarged as indicated by the enlarged region 411b within the image space. Light rays passing through the region of interest 411a are redirected to reflect an enlarged region 411b, whereas those light rays passing through the actual enlarged region 411b but not through the region of interest 411a, for example, light ray 413, are not redirected. Light such as from light ray 413 can be either rendered transparently or else not rendered at all.

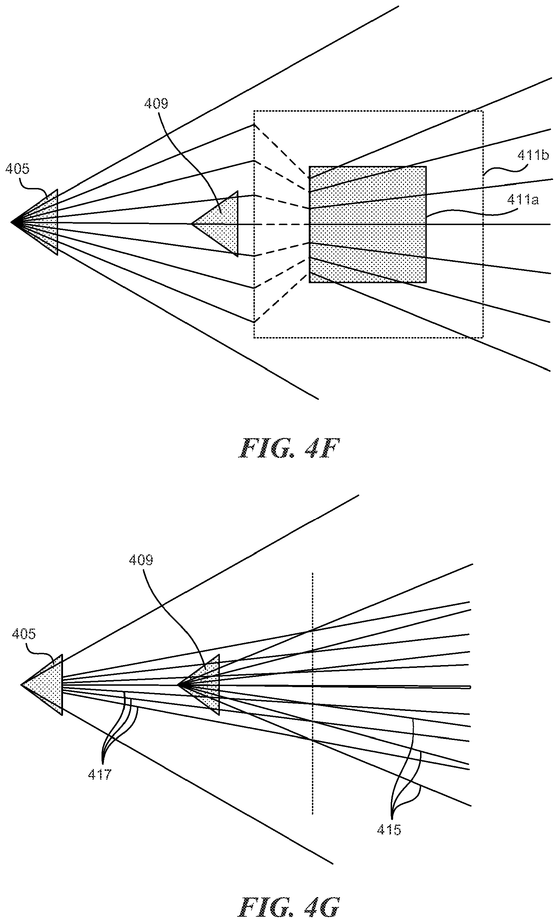

This same enlargement technique is illustrated in Figures 4F and 4G as the rendering of a virtual camera 409 closer to the region 411a. By rendering the close virtual camera 409, the region 411a is enlarged to encompass the area of region 411b. Figure 4G illustrates both this enlargement (indicated by light rays 415) and a conventional zoom (indicated by light rays 417). As shown in Figure 4G, enlargement and zoom are the same at the focal plane 419, but zoomed objects have incorrect foreshortening.

Enlarged volumes can be fixed to the position in space, rather than a particular angular area of a view. For example, a tumor or other portion of the surgical site can be enlarged, and as the user moves her head while wearing the head-mounted display assembly 100, the image can be manipulated such that the area of enlargement remains fixed to correspond to the physical location of the tumor. In some embodiments, the regions "behind" the enlarged area can be rendered transparently so that the user can still perceive that area that is being obscured by the enlargement of the area of interest.

In some embodiments, the enlarged volume does not need to be rendered at its physical location, but rather can be positioned independently from the captured volume. For example, the enlarged view can be rendered closer to the surgeon and at a different angle. In some embodiments, the position of external tools can be tracked for input. For example, the tip of a scalpel or other surgical tool can be tracked (e.g., using the tracker 203), and the enlarged volume can be located at the tip of the scalpel or other surgical tool. In some embodiments, the surgical tool can include haptic feedback or physical controls for the system or other surgical systems. In situations in which surgical tools are controlled electronically or electromechanically (e.g., during telesurgery where the tools are controlled with a surgical robot), the controls for those tools can be modified depending on the visualization mode. For example, when the tool is disposed inside the physical volume to be visually transformed (e.g., enlarged), the controls for the tool can be modified to compensate for the visual scaling, rotation, etc. This allows for the controls to remain the same inside the visually transformed view and the surrounding view. This modification of the tool control can aid surgeons during remote operation to better control the tools even as visualization of the tools and the surgical site are modified.

Information from additional cameras in the environment located close to points of interest can be fused with images from the imagers coupled to the head-mounted display, thereby improving the ability to enlarge regions of interest. Depth information can be generated or gained from a depth sensor and used to bring the entirety of the scene into focus by co-locating the focal plane with the physical geometry of the scene. As with other mediated reality, data can be rendered and visualized in the environment. The use of light fields can allow for viewing around occlusions and can remove specular reflections. In some embodiments, processing of light fields can also be used to increase the contrast between tissue types.

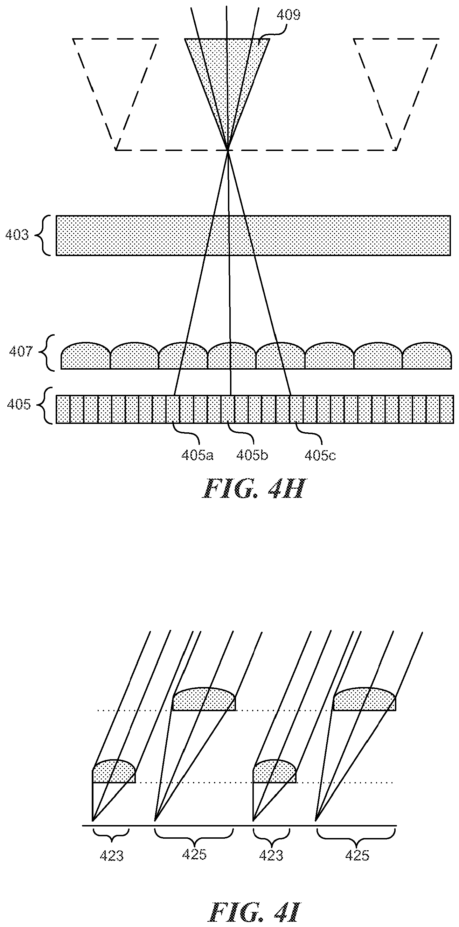

Figure 4H illustrates selective activation of sensor elements 405n depending on the virtual camera 409 being rendered. As illustrated, only sensor elements 405a-c of the array of the sensor elements are needed to render the virtual camera 409. Accordingly, the other sensor elements can be deactivated. This reduces required power and data by not capturing and transmitting unused information.

Figure 4I illustrates an alternative configuration of a lenslet array 421 for a plenoptic camera. As illustrated, a first plurality of lenslets 423 has a first curvature and is spaced at a first distance from the image sensor, and a second plurality of lenslets 425 has a second curvature and is spaced at a second distance from the image sensor. In this embodiment, the first plurality of lenslets 423 and the second plurality of lenslets 425 are interspersed. In other embodiments, the first plurality of lenslets 423 can be disposed together, and the second plurality of lenslets 425 can also be disposed together but separated from the first plurality of lenslets. By varying the arrangement and type of lenslets in the array, angular and spatial resolution can be varied.

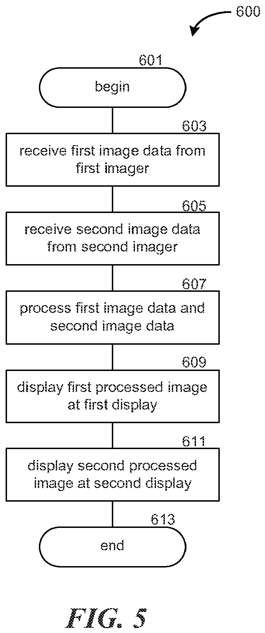

Figure 5 is a block diagram of a method for providing a mediated-reality display for surgical visualization according to one embodiment of the present technology. The routine 600 begins in block 601. In block 603, first image data is received from a first imager 113a, and in block 605 second image data is received from a second imager 113b. For example, the first imager 113a can be positioned over a user's right eye when wearing a head-mounted display assembly, and the second imager 113b can be positioned over the user's left eye when wearing the head-mounted display assembly 100. The routine 600 continues in block 607 with processing the first image data and the second image data. The processing can be performed by remote electronics (e.g., computing component 207) in wired or wireless communication with the head-mounted display assembly 100. Or in some embodiments, the processing can be performed via control electronics 115a-b carried by the assembly 100. In block 609, the first processed image is displayed at a first display 119a, and in block 611 a second processed image is displayed at a second display 119b. The first display 119a can be configured to display the first processed image to the user's right eye when wearing the assembly 100, and the second display 119b can be configured to display the second processed image to the user's left eye when wearing the assembly 100. The first and second processed images can be presented for stereoscopic effect, such that the user perceives a three-dimensional depth of field when viewing both processed images simultaneously.

Although several embodiments described herein are directed to mediated-reality visualization systems for surgical applications, other uses of such systems are possible. For example, a mediated-reality visualization system including a head-mounted display assembly with an integrated display device and an integrated image capture device can be used in construction, manufacturing, the service industry, gaming, entertainment, and a variety of other contexts.

### Conclusion

The above detailed descriptions of embodiments of the technology are not intended to be exhaustive or to limit the technology to the precise form disclosed above. Although specific embodiments of, and examples for, the technology are described above for illustrative purposes, various equivalent modifications are possible within the scope of the technology, as those skilled in the relevant art will recognize. For example, while steps are presented in a given order, alternative embodiments may perform steps in a different order. The various embodiments described herein may also be combined to provide further embodiments.

From the foregoing, it will be appreciated that specific embodiments of the invention have been described herein for purposes of illustration, but well-known structures and functions have not been shown or described in detail to avoid unnecessarily obscuring the description of the embodiments of the technology. Where the context permits, singular or plural terms may also include the plural or singular term, respectively.

Moreover, unless the word "or" is expressly limited to mean only a single item exclusive from the other items in reference to a list of two or more items, then the use of "or" in such a list is to be interpreted as including (a) any single item in the list, (b) all of the items in the list, or (c) any combination of the items in the list. Additionally, the term "comprising" is used throughout to mean including at least the recited feature(s) such that any greater number of the same feature and/or additional types of other features are not precluded. It will also be appreciated that specific embodiments have been described herein for purposes of illustration, but that various modifications may be made without deviating from the technology. Further, while advantages associated with certain embodiments of the technology have been described in the context of those embodiments, other embodiments may also exhibit such advantages, and not all embodiments need necessarily exhibit such advantages to fall within the scope of the technology. Accordingly, the disclosure and associated technology can encompass other embodiments not expressly shown or described herein.

## Claims

1. A mediated-reality system for surgical visualization, comprising:
a head-mounted display assembly (100) comprising:
a frame (103) configured to be worn on a user's head;
an image capture device (113a, 113b) coupled to the frame (103), the image capture device (113a, 113b) is a plenoptic camera including an image sensor (405), and an array of sensor elements (405a-d) corresponding to different portions of the image sensor (405) that receive light from different lenslets (407), each portion of the sensor elements (405a-d) having a different field of view;
a display device (109) coupled to the frame (103), the display device (109) configured to display an image towards an eye of the user;
a computing device (207) in communication with the display device (109) and the image capture device (113a, 113b), the computing device (207) configured to:
receive image data from the image capture device (113a, 113b);
render a virtual camera (409) having a position and a field of view, and wherein only data from a portion of sensor elements (405a-d) for which the position of the virtual camera (409) falls within the field of view of the portion of sensor element (405a-d), or for which the field of view of the portion of sensor element (405a-d) is within the field of view of the virtual camera (409), is used to render the image from the virtual camera (409), wherein the virtual camera (409) may be positioned in front of the sensor elements (405a-d), or the virtual camera (409) may be positioned behind the sensor elements (405a-d); and
present an image corresponding to the virtual camera (409) from the image data via the display device (109).

2. The mediated-reality visualization system of claim 1 wherein the computing device (207) is configured to present the image in real time.

3. The mediated-reality visualization system of claim 1, further comprising at least a second image capture (203) device spaced apart from the head-mounted display assembly (100), wherein the computing device (207) is further configured to:
receive additional image data from the second image capture device (203); and
present an image at least in part from the additional image data via the display device (109).

4. The mediated-reality visualization system of claim 1 wherein the image capture device (113a, 113b) comprises at least one plenoptic camera.

5. The mediated-reality visualization system of any preceding claim wherein the computing device (207) is configured to render the at least one virtual camera (409) at a location corresponding to a position of a user's eye when the frame is worn by the user.

6. The mediated-reality visualization system of any preceding claim, wherein rendering the at least one virtual camera (409) comprises rendering an enlarged view of a portion of a captured light field.

7. The mediated-reality visualization system of any preceding claim, wherein the position of the virtual camera (409) is in front of the sensor elements (405a-d), and wherein only data from portions of sensor elements (405a-d) for which the position of the virtual camera (409) falls within the field of view of said portions of sensor element (405a-d) is used to render the image from the virtual camera (409).

8. The mediated-reality visualization system of any one of claims 1 to 6, wherein the position of the virtual camera (409) is behind the sensor elements (405a-d), and wherein only data from portions of sensor elements (405a-d) for which the field of view of said portions of sensor element (405a-d) is within the field of view of the virtual camera (409) is used to render the image from the virtual camera (409).

9. A method for providing mediated-reality surgical visualization, the method comprising:
providing a head-mounted display (100) comprising a frame (103) configured to be mounted to a user's head, first and second imagers (113a, 113b) coupled to the frame, and first and second displays (119a, 119b) coupled to the frame (103);
receiving first image data from the first imager (113a), being a plenoptic camera which includes a first image sensor (405), and an array of first sensor elements (405a-d) corresponding to different portions of the first image sensor (405) that receive light from different lenslets (407), each portion of the first sensor elements (405a-d) having a different field of view;
receiving second image data from the second imager (113b), being a plenoptic camera which includes a second image sensor (405), and an array of second sensor elements (405a-d) corresponding to different portions of the second image sensor (405) that receive light from different lenslets (407), each portion of the second sensor elements (405a-d) having a different field of view;
processing the first image data and the second image data;
rendering a first virtual camera (409) having a position and a field of view, and wherein only data from a portion of first sensor elements (405a-d) for which the position of the first virtual camera (409) falls within the field of view of the portion of first sensor element (405a-d), or for which the field of view of the portion of first sensor elements (405a-d) is within the field of view of the first virtual camera (409), is used to render the image from the first virtual camera (409), wherein the first virtual camera (409) may be positioned in front of the sensor elements (405a-d), or the first virtual camera (409) may be positioned behind the sensor elements (405a-d);
displaying an image corresponding to the virtual camera (409) from the first processed image data at the first display (119a);
rendering a second virtual camera (409) having a position and a field of view, and wherein only data from a portion of second sensor elements (405a-d) for which the position of the second virtual camera (409) falls within the field of view of the portion of second sensor element (405a-d), or for which the field of view of the portion of second sensor element (405a-d) is within the field of view of the second virtual camera (409), is used to render the image from the second virtual camera (409), wherein the second virtual camera (409) may be positioned in front of the sensor elements (405a-d), or the second virtual camera (409) may be positioned behind the sensor elements (405a-d); and
displaying an image corresponding to the second virtual camera from the second processed image data at the second display (119b).

10. The method of claim 9 wherein the first and second processed image data are displayed at the first and second displays (119a, 119b) in real time.

11. The method of claim 9, further comprising:
receiving third image data;
processing the third image data; and
displaying the processed third image data at the first display (119a) and/or second display (119b).

12. The method of claim 11 wherein the third image data comprises at least one of: fluorescence image data, magnetic resonance imaging data; computed tomography image data, X-ray image data, anatomical diagram data, or vital-signs data.

13. The method of claim 11 wherein the third image data is received from a third imager (203) spaced apart from the head-mounted display (100).

14. The method of claim 9, further comprising tracking movement of the head-mounted display (100).

15. The method of claim 9 wherein rendering the at first and second virtual cameras (409) comprises rendering the at first and second virtual cameras at locations corresponding to a position of the user's eyes when the display (100) is mounted to a user's head.

## Patentansprüche

1. System vermittelter Realität zur chirurgischen Visualisierung, Folgendes umfassend:
eine Kopfanzeigebaugruppe(100), Folgendes umfassend:
ein Gestell (103), das dafür konfiguriert ist, am Kopf eines Benutzers getragen zu werden,
ein Bilderfassungsgerät (113a, 113b), das an das Gestell (103) gekoppelt ist, wobei das Bilderfassungsgerät (113a, 113b) eine plenoptische Kamera ist, die einen Bildsensor (405) und eine Anordnung von Sensorelementen (405a-d) beinhaltet, die verschiedenen Abschnitten des Bildsensors (405) entsprechen, die Licht von verschiedenen Lenslets (407) empfangen, wobei jeder Abschnitt der Sensorelemente (405a-d) ein anderes Sichtfeld aufweist,
ein Anzeigegerät (109), das an das Gestell (103) gekoppelt ist, wobei das Anzeigegerät (109) dafür konfiguriert ist, ein Bild hin zu einem Auge des Benutzers anzuzeigen,
ein Rechengerät (207) in Verbindung mit dem Anzeigegerät (109) und dem Bilderfassungsgerät (113a, 113b), wobei das Rechengerät (207) für Folgendes konfiguriert ist,:
Empfangen von Bilddaten von dem Bilderfassungsgerät (113a, 113b),
Rendern einer virtuellen Kamera (409), die eine Position und ein Sichtfeld aufweist, und wobei nur Daten von einem Abschnitt von Sensorelementen (405a-d), für die die Position der virtuellen Kamera (409) mit dem Sichtfeld des Abschnitts des Sensorelements (405a-d) zusammenfällt oder für die das Sichtfeld des Abschnitts des Sensorelements(405a-d) in dem Sichtfeld der virtuellen Kamera (409) liegt, verwendet werden, um das Bild von der virtuellen Kamera (409) zu rendern, wobei die virtuelle Kamera (409) vor den Sensorelementen (405a-d) positioniert sein kann oder die virtuelle Kamera (409) hinter den Sensorelementen (405a-d) positioniert sein kann, und
Darstellen eines Bildes, das der virtuellen Kamera (409) entspricht, aus den Bilddaten mittels des Anzeigegeräts (109).

2. System vermittelter Realität zur Visualisierung nach Anspruch 1, wobei das Rechengerät (207) dafür konfiguriert ist, das Bild in Echtzeit darzustellen.

3. System vermittelter Realität zur Visualisierung nach Anspruch 1, ferner mindestens ein zweites Bilderfassungsgerät (203) umfassend, das von der Kopfanzeigebaugruppe (100) beabstandet ist, wobei das Rechengerät (207) ferner für Folgendes konfiguriert ist:
Empfangen zusätzlicher Bilddaten von dem zweiten Bilderfassungsgerät (203) und
Darstellen eines Bildes zumindest teilweise aus den zusätzlichen Bilddaten mittels des Anzeigegeräts (109).

4. System vermittelter Realität zur Visualisierung nach Anspruch 1, wobei das Bilderfassungsgerät (113a, 113b) mindestens eine plenoptische Kamera umfasst.

5. System vermittelter Realität zur Visualisierung nach einem der vorhergehenden Ansprüche, wobei das Rechengerät dafür konfiguriert ist, die mindestens eine virtuelle Kamera (409) an einer Stelle zu rendern, die einer Position eines Benutzerauges entspricht, wenn das Gestell von dem Benutzer getragen wird.

6. System vermittelter Realität zur Visualisierung nach einem der vorhergehenden Ansprüche, wobei das Rendern der mindestens einen virtuellen Kamera (409) das Rendern einer vergrößerten Ansicht eines Abschnitts eines erfassten Lichtfeldes umfasst.

7. System vermittelter Realität zur Visualisierung nach einem der vorhergehenden Ansprüche, wobei die Position der virtuellen Kamera (409) vor den Sensorelementen (405a-d) liegt und wobei nur Daten von Abschnitten von Sensorelementen (405a-d), für die die Position der virtuellen Kamera (409) mit dem Sichtfeld der Abschnitte des Sensorelements (405a-d) zusammenfällt, verwendet werden, um das Bild von der virtuellen Kamera (409) zu rendern.

8. System vermittelter Realität zur Visualisierung nach einem der Ansprüche 1 bis 6, wobei die Position der virtuellen Kamera (409) hinter den Sensorelementen (405a-d) liegt und wobei nur Daten von Abschnitten von Sensorelementen (405a-d), für die das Sichtfeld der Abschnitte des Sensorelements (405a-d) im Sichtfeld der virtuellen Kamera (409) liegt, verwendet werden, um das Bild von der virtuellen Kamera (409) zu rendern.

9. Verfahren zum Bereitstellen chirurgischer Visualisierung mittels vermittelter Realität, wobei das Verfahren Folgendes umfasst:
Bereitstellen eines Kopfanzeigegeräts(100), das ein Gestell (103), das dafür konfiguriert ist, am Kopf eines Benutzers angebracht zu werden, ein erstes und ein zweites Bildgerät (113a, 113b), die an das das Gestell (103) gekoppelt sind, und eine erste und eine zweite Anzeige (119a, 119b), die an das Gestell (103) gekoppelt sind, umfasst,
Empfangen erster Bilddaten von dem ersten Bildgerät (113a), das eine plenoptische Kamera ist, die einen ersten Bildsensor (405) und eine Anordnung erster Sensorelemente (405a-d) beinhaltet, die verschiedenen Abschnitten des ersten Bildsensors (405) entsprechen, die Licht von verschiedenen Lenslets (407) empfangen, wobei jeder Abschnitt der ersten Sensorelemente (405a-d) ein anderes Sichtfeld aufweist,
Empfangen zweiter Bilddaten von dem zweiten Bildgerät (113b), das eine plenoptische Kamera ist, die einen zweiten Bildsensor (405) und eine Anordnung zweiter Sensorelemente (405a-d) beinhaltet, die verschiedenen Abschnitten des zweiten Bildsensors (405) entsprechen, die Licht von verschiedenen Lenslets (407) empfangen, wobei jeder Abschnitt der zweiten Sensorelemente (405a-d) ein anderes Sichtfeld aufweist,
Verarbeiten der ersten Bilddaten und der zweiten Bilddaten,
Rendern einer ersten virtuellen Kamera (409), die eine Position und ein Sichtfeld aufweist, und wobei nur Daten von einem Abschnitt erster Sensorelemente (405a-d), für die die Position der ersten virtuellen Kamera (409) mit dem Sichtfeld des Abschnitts der ersten Sensorelemente (405a-d) zusammenfällt oder für die das Sichtfeld des Abschnitts der ersten Sensorelemente(405a-d) in dem Sichtfeld der ersten virtuellen Kamera (409) liegt, verwendet werden, um das Bild von der ersten virtuellen Kamera (409) zu rendern, wobei die erste virtuelle Kamera (409) vor den Sensorelementen (405a-d) positioniert sein kann oder die erste virtuelle Kamera (409) hinter den Sensorelementen (405a-d) positioniert sein kann,
Anzeigen eines Bildes, das der virtuellen Kamera (409) entspricht, aus den ersten verarbeiteten Bilddaten auf der ersten Anzeige (119a),
Rendern einer zweiten virtuellen Kamera (409), die eine Position und ein Sichtfeld aufweist, und wobei nur Daten von einem Abschnitt zweiter Sensorelemente (405a-d), für die die Position der zweiten virtuellen Kamera (409) mit dem Sichtfeld des Abschnitts der zweiten Sensorelemente (405a-d) zusammenfällt oder für die das Sichtfeld des Abschnitts des zweiten Sensorelements(405a-d) in dem Sichtfeld der zweiten virtuellen Kamera (409) liegt, verwendet werden, um das Bild von der zweiten virtuellen Kamera (409) zu rendern, wobei die zweite virtuelle Kamera (409) vor den Sensorelementen (405a-d) positioniert sein kann oder die zweite virtuelle Kamera (409) hinter den Sensorelementen (405a-d) positioniert sein kann, und
Anzeigen eines Bildes, das der zweiten virtuellen Kamera (409) entspricht, aus den zweiten verarbeiteten Bilddaten auf der zweiten Anzeige (119b).

10. Verfahren nach Anspruch 9, wobei die ersten und die zweiten verarbeiteten Bilddaten auf der ersten und der zweiten Anzeige (119a, 119b) in Echtzeit angezeigt werden.

11. Verfahren nach Anspruch 9, ferner Folgendes umfassend:
Empfangen dritter Bilddaten,
Verarbeiten der dritten Bilddaten und
Anzeigen der verarbeiteten dritten Bilddaten auf der ersten Anzeige (119a) und/oder der zweiten Anzeige (119b).

12. Verfahren nach Anspruch 11, wobei die dritten Bilddaten mindestens eines des Folgenden umfassen: Fluoreszenzbilddaten, Magnetresonanzbilddaten, Computertomografiebilddaten, Röntgenbilddaten, Anatomiedarstellungsdaten oder Vitalzeichendaten .

13. Verfahren nach Anspruch 11, wobei die dritten Bilddaten von einem dritten Bildgerät (203) empfangen werden, das von dem Kopfanzeigegerät (100) beabstandet ist.

14. Verfahren nach Anspruch 9, ferner das Nachführen der Bewegung der Kopfanzeigebaugruppe (100) umfassend.

15. Verfahren nach Anspruch 9, wobei das Rendern der ersten und der zweiten virtuellen Kamera (409) das Rendern der ersten und der zweiten Kamera an Stellen umfasst, die einer Position der Benutzeraugen entspricht, wenn die Anzeige (100) am Kopf eines Benutzers angebracht ist.

## Revendications

1. Système de visualisation chirurgicale par réalité induite, comprenant :
un ensemble d'affichage au mécanisme monté sur la tête (100) comportant :
une monture (103) configurée pour être portée sur la tête d'un utilisateur ;
un dispositif de capture d'images (113a, 113b) couplé à la monture (103), le dispositif de capture d'images (113a, 113b) étant une caméra plénoptique intégrant un capteur d'images (405) et un réseau d'éléments capteurs (405a-d) correspondant à différentes parties du capteur d'images (405) qui reçoivent la lumière émise par différentes lentilles (407), chaque partie des éléments capteurs (405a-d) ayant un différent champ de vision ;
un dispositif d'affichage (109) couplé à la monture (103), le dispositif d'affichage (109) étant configuré pour afficher une image en direction d'un œil de l'utilisateur ;
un dispositif informatique (207) en communication avec le dispositif d'affichage (109) et le dispositif de capture d'images (113a, 113b), le dispositif informatique (207) étant configuré pour :
recevoir des données d'image en provenance du dispositif de capture d'images (113a, 113b) ;
définir un rendu avec une caméra virtuelle (409) ayant un positionnement et un champ de vision, et dans lequel seules des données d'une partie des éléments capteurs (405a-d) pour lesquelles le positionnement de la caméra virtuelle (409) se situe dans le champ de vision de la partie des éléments capteurs (405a-d), ou pour lesquelles le champ de vision de la partie des éléments capteurs (405a-d) se situe dans le champ de vision de la caméra virtuelle (409), sont utilisées pour définir un rendu d'image à partir de la caméra virtuelle (409), dans lequel il se peut que la caméra virtuelle (409) soit positionnée devant les éléments capteurs (405a-d), ou que la caméra virtuelle (409) soit positionnée derrière les éléments capteurs (405ad) ; et
présenter une image correspondant à la caméra virtuelle (409) à partir des données d'image sur le dispositif d'affichage (109).

2. Système de visualisation chirurgicale par réalité induite selon la revendication 1, dans lequel le dispositif informatique (207) est configuré de sorte à présenter l'image en temps réel.

3. Système de visualisation chirurgicale par réalité induite selon la revendication 1, comprenant en outre au moins un second dispositif de capture d'images (203) situé à distance de l'ensemble d'affichage au mécanisme monté sur la tête (100), dans lequel le dispositif informatique (207) est en outre configuré de sorte à :
recevoir des données d'image supplémentaires en provenance du second dispositif de capture d'images (203) ; et présenter une image au moins en partie à partir des données d'images supplémentaires sur le dispositif d'affichage (109).

4. Système de visualisation chirurgicale par réalité induite selon la revendication 1, dans lequel le dispositif de capture d'images (113a, 113b) comprend au moins une caméra plénoptique.

5. Système de visualisation chirurgicale par réalité induite selon l'une quelconque des revendications précédentes, dans lequel le dispositif informatique (207) est configuré pour définir un rendu avec au moins une caméra virtuelle (409) à un emplacement correspondant à un positionnement de l'œil d'un utilisateur lorsque ce dernier porte la monture.

6. Système de visualisation chirurgicale par réalité induite selon l'une quelconque des revendications précédentes, dans lequel le rendu avec au moins une caméra virtuelle (409) comprend le rendu d'une vision élargie d'une partie d'un champ lumineux capturé.

7. Système de visualisation chirurgicale par réalité induite selon l'une quelconque des revendications précédentes, dans lequel le positionnement de la caméra virtuelle (409) se situe devant les éléments capteurs (405a-d), et dans lequel seules des données des parties des éléments capteurs (405a-d), pour lesquelles le positionnement de la caméra virtuelle (409) se situe dans le champ de vision desdites parties des éléments capteurs (405a-d), sont utilisées pour définir un rendu d'image à partir de la caméra virtuelle (409).

8. Système de visualisation chirurgicale par réalité induite selon l'une quelconque des revendications 1 à 6, dans lequel le positionnement de la caméra virtuelle (409) se situe derrière les éléments capteurs (405a-d), et dans lequel seules des données des parties des éléments capteurs (405a-d) pour lesquelles le champ de vision desdites parties des éléments capteurs (405a-d) se situe dans le champ de vision de la caméra virtuelle (409), sont utilisées pour définir un rendu d'image à partir de la caméra virtuelle (409).

9. Procédé de visualisation chirurgicale par réalité induite, le procédé consistant à :
fournir un afficheur au mécanisme monté sur la tête (100) comportant une monture (103) configurée pour être portée sur la tête d'un utilisateur, un premier et un second imageurs (113a, 113b) couplés à la monture (103), et un premier et un second afficheurs (119a, 119b) couplés à la monture (103) ;
recevoir des premières données d'image en provenance du premier imageur (113a), qui est une caméra plénoptique intégrant un premier capteur d'images (405), et un réseau de premiers éléments capteurs (405a-d) correspondant à différentes parties du premier capteur d'images (405) qui reçoivent la lumière émise par différentes lentilles (407), chaque partie des premiers éléments capteurs (405a-d) ayant un différent champ de vision ;
recevoir des secondes données d'image en provenance du second imageur (113b), qui est une caméra plénoptique intégrant un second capteur d'images (405), et un réseau de seconds éléments capteurs (405a-d) correspondant à différentes parties du second capteur d'images (405) qui reçoivent la lumière émise par différentes lentilles (407), chaque partie des seconds éléments capteurs (405a-d) ayant un différent champ de vision ;
traiter les premières données d'image et les secondes données d'image ;
définir un rendu avec une première caméra virtuelle (409) ayant un positionnement et un champ de vision, et dans lequel seules des données d'une partie des premiers éléments capteurs (405a-d) pour lesquelles le positionnement de la première caméra virtuelle (409) se situe dans le champ de vision de la partie des premiers éléments capteurs (405a-d), ou pour lesquelles le champ de vision de la partie des premiers éléments capteurs(405a-d) se situe dans le champ de vision de la première caméra virtuelle (409), sont utilisées pour définir un rendu d'image à partir de la première caméra virtuelle (409), dans lequel il se peut que la première caméra virtuelle (409) soit positionnée devant les éléments capteurs (405a-d), ou que la première caméra virtuelle (409) soit positionnée derrière les éléments capteurs (405a-d) ;
afficher une image correspondant à la caméra virtuelle (409) à partir des premières données d'image traitées sur le premier afficheur (109) ;
définir un rendu avec une seconde caméra virtuelle (409) ayant un positionnement et un champ de vision, et dans lequel seules des données d'une partie des seconds éléments capteurs (405a-d) pour lesquelles le positionnement de la seconde caméra virtuelle (409) se situe dans le champ de vision de la partie des seconds éléments capteurs (405a-d), ou pour lesquelles le champ de vision de la partie des seconds éléments capteurs(405a-d) se situe dans le champ de vision de la seconde caméra virtuelle (409), sont utilisées pour définir un rendu d'image à partir de la seconde caméra virtuelle (409), dans lequel il se peut que la seconde caméra virtuelle (409) soit positionnée devant les éléments capteurs (405a-d), ou que la seconde caméra virtuelle (409) soit positionnée derrière les éléments capteurs (405a-d) ; et
afficher une image correspondant à la seconde caméra virtuelle (409) à partir des secondes données d'image traitées sur le second afficheur (109).

10. Procédé selon la revendication 9, dans lequel les premières et les secondes données d'image traitées sont affichées sur le premier et le second afficheurs (119a, 110b) en temps réel.

11. Procédé selon la revendication 9, consistant à :
recevoir des troisièmes données d'image ;
traiter les troisièmes données d'image ; et
afficher les troisièmes données traitées sur le premier afficheur (119a) et/ou sur le second afficheur (119b).

12. Procédé selon la revendication 11, dans lequel les troisièmes données d'image comprennent au moins : des données d'image de fluorescence, des données obtenues par imagerie par résonance magnétique, des données d'image de tomographies assistée par ordinateur, des données d'une image à rayons x, des données de diagramme anatomique, ou des données sur les signes vitaux.

13. Procédé selon la revendication 11, dans lequel les troisièmes données d'image proviennent d'un troisième imageur (203) situé à distance de l'afficheur au mécanisme monté sur la tête (100).

14. Procédé selon la revendication 9, consistant en outre à suivre le mouvement de l'afficheur au mécanisme monté sur la tête (100).

15. Procédé selon la revendication 9, dans lequel le rendu avec la première et la seconde caméras virtuelles (409) comprend le rendu avec la première et la seconde caméras virtuelles à des emplacements correspondant à un positionnement des yeux de l'utilisateur lorsque l'afficheur (100) est monté sur la tête de l'utilisateur.
